Europäisches Patentamt

European Patent Office

Office européen des brevets

(19)

(11) Veröffentlichungsnummer: **0 180 998**
**B1**

(12)

# EUROPÄISCHE PATENTSCHRIFT

(45) Veröffentlichungstag der Patentschrift:
01.03.89

(21) Anmeldenummer: 85114211.7

(22) Anmeldetag: 07.11.85

(51) Int. Cl.⁴: **C 07 C 19/045**, C 07 C 17/38,
C 07 C 21/06, C 07 C 17/34

(54) Verfahren zur Aufbereitung von 1.2-Dichlorethan für die Pyrolyse.

(30) Priorität: 09.11.84 DE 3441045

(43) Veröffentlichungstag der Anmeldung:
14.05.86 Patentblatt 86/20

(45) Bekanntmachung des Hinweises auf die Patenterteilung:
01.03.89 Patentblatt 89/9

(84) Benannte Vertragsstaaten:
BE DE FR GB IT NL SE

(56) Entgegenhaltungen:
EP-A- 0 002 501

(73) Patentinhaber: WACKER-CHEMIE GMBH,
Prinzregentenstrasse 22, D-8000 München 22 (DE)

(72) Erfinder: Dummer, Gerhard, Dipl.-Ing. (TU),
Lohnerstrasse 12, D-8269 Burgkirchen (DE)
Erfinder: Haselwarter, Klaus, Dipl.-Ing. (FH),
Hauptstrasse 15b, D-8261 Emmerting (DE)
Erfinder: Hirschmann, Peter, Dipl.-Ing. (FH), Jägerweg 5,
D-8263 Burghausen (DE)
Erfinder: Schmidhammer, Ludwig, Dr. Dipl.-Chem.,
Pappelweg 5, D-8261 Haiming (DE)
Erfinder: Strasser, Rudolf, Dr. Dipl.-Chem.,
Lindacherstrasse 58, D-8263 Burghausen (DE)

## Beschreibung

Die Erfindung betrifft ein Verfahren zur Aufbereitung von 1,2-Dichlorethan für die Pyrolyse zu Vinylchlorid und Chlorwasserstoff, wobei

a) 1,2-Dichlorethan verwendet wird, das zu einem Anteil von 35 bis 55 Gew.-% solches 1,2-Dichlorethan enthält, das bei der Pyrolyse nicht umgesetzt und zur Pyrolyse zurückgeführt wurde,

b) die Pyrolyse bei Drücken von 10 bis 15 bar abs. und Temperaturen von 480 bis 540 °C durchgeführt wird,

c) 1,2-Dichlorethan vor Eintritt in die Spaltzone des Pyrolyseofens mittels Wasserdampf von 16 bis 24 bar abs. vorverdampft wird,

d) das den Spaltofen verlassende Pyrolyseprodukt zunächst durch Abtrennen von Chlorwasserstoff und Vinylchlorid aufgearbeitet wird,

e) verbleibendes, nicht umgesetztes Dichlorethan durch Umsetzen mit Chlor in Flüssigphase bei Temperaturen von 0 bis 80 °C in Gegenwart von hydroxylgruppenhaltigen Aromaten von Leichtsiedern befreit wird.

Vinylchlorid wird bekanntlich durch Pyrolyse von 1,2-Dichlorethan gewonnen. Innerhalb grosstechnischer Produktionsverfahren wird dabei 1,2-Dichlorethan verwendet, das im wesentlichen aus drei verschiedenen Quellen stammt, nämlich aus der Direktchlorierung von Ethylen mit Chlor, aus der Oxichlorierung von Ethylen mit Chlorwasserstoff und Sauerstoff, sowie aus der Aufarbeitungsstufe des 1,2-Dichlorethan-Pyrolyseprodukts, das je nach Spaltrate noch etwa 40 bis 50% nicht umgesetzten 1,2-Dichlorethans enthält.

1,2-Dichlorethan sollte als Einsatzstoff für die Pyrolyse in möglichst reiner Form verwendet werden, um einer erhöhten Nebenproduktbildung einschliesslich erhöhter Koksbildung vorzubeugen und ferner die Kinetik der Spaltungsreaktion nicht zu beeinträchtigen.

Üblicherweise wird Dichlorethan, das aus einer der vorstehend genannten Quellen stammt, deshalb, bevor es einem Pyrolyseprozess unterworfen wird, zunächst gewaschen, um Katalysator und ggf. saure Bestandteile zu entfernen und anschliessend einer Destillationsstrasse zur Entfernung von Leicht- und Hochsiedern aufgegeben.

Während nun die Abtrennung von Hochsiedern in der Praxis störungsfrei verläuft, bereitet die Leichtsiederabtrennung verfahrenstechnische Schwierigkeiten, die zu häufigen Unterbrechungen des kontinuierlichen Verfahrensablaufs führen.

Bei den Leichtsiedern, die durch nicht umgesetztes 1,2-Dichlorethan aus der Pyrolyse eingeschleppt werden, handelt es sich im wesentlichen um Benzol, 2-Chlorbutadien-1,3(Chloropren), 1,1-Dichlorethylen, cis- und trans-1,2-Dichlorethylen, 1,1-Dichlorethan, Ethylchlorid, Chloroform und Tetrachlorkohlenstoff. Wegen der geringen Siedepunktsunterschiede sind zur destillativen Abtrennung dieser Leichtsieder Kolonnen mit hoher Trennleistung bei gleichzeitig hohem Rücklaufverhältnis erforderlich. Die quantitative Entfernung der Leichtsieder ist daher sehr energieaufwendig. Chloropren erschwert das Destillationsverfahren zudem durch seine Neigung zur Polymerisatbildung.

Nun ist bereits gemäss DE-AS 12 42 594 ein Verfahren zur Aufbereitung von 1,2-Dichlorethan für die Pyrolyse bekanntgeworden, bei dem auf die Abtrennung von Leichtsiedern verzichtet wird. Das eingesetzte 1,2-Dichlorethan wird lediglich von Hochsiedern befreit, mit reinem 1,2-Dichlorethan aus anderen Quellen vermischt und so zur Pyrolyseeinheit zurückgeführt. Dieses Verfahren lässt sich jedoch ausschliesslich unter besonders massiven Pyrolysebedingungen durchführen. Es muss bei Drücken von 20 bis 35 bar abs. und einem entsprechend hohen Temperaturniveau verdampft werden. Unter den sich bereits notwendigerweise im Vorverdampferteil der Pyrolyse ergebenden massiven Bedingungen zersetzen sich Leichtsieder, wie Chloropren, Tetrachlorkohlenstoff, Chloroform, Ethylchlorid bereits vor Eintritt in den Strahlungsteil des Spaltofens, in dem die 1,2-Dichlorethanpyrolyse an sich stattfindet. Letztlich stellt sich ein Gleichgewicht zwischen Leichtsiederzersetzung und erneuter Leichtsiederbildung ein, die einen kontinuierlichen Betrieb gestatten.

Nachteiligerweise müssen bei dieser Arbeitsweise jedoch eine erhöhte Bildung von Koks als Zersetzungsprodukt der Leichtsieder bereits im Verdampfungsteil der Spaltanlage in Kauf genommen werden, was die Standzeit derartiger Vorrichtungen erheblich vermindert. Darüberhinaus neigt auch 1,2-Dichlorethan unter den gegebenen hohen Temperaturbeanspruchungen zu Polykondensation unter Ausbildung teeriger Ablagerungen.

Nun kann die Pyrolyse von 1,2-Dichlorethan mit Vorteil unter weit schonenderen Bedingungen durchgeführt werden – etwa im Druckbereich von 10 bis 15 bar abs. und Temperaturen von 480 bis 540 °C. Bei dieser Arbeitsweise kann 1,2-Dichlorethan schonend durch Wärmetausch mit technisch üblicherweise zur Verfügung stehendem Wasserdampf in den gasförmigen Zustand übergeführt werden. Die Nebenproduktbildung wird dadurch deutlich gemindert. Andererseits gelangen unter diesen Bedingungen die Leichtsieder, mit denen eingesetztes 1,2-Dichlorethan verunreinigt ist, in die eigentliche Spaltzone.

Chloropren vermindert als Radikalfänger dabei die Spaltrate des Prozesses. Ethylchlorid verursacht die Bildung von 1,3-Butadien, Allen und Methylacetylen, die sich als Polymerisationshemmer im produzierten Vinylchlorid wiederfinden. Insbesondere Allen und Methylacetylen stören bereits im unteren einstelligen ppm-Bereich.

Nun ist auch bereits bekannt, einen Teil der Leichtsieder durch Chlorieren in Hochsieder umzuwandeln. Dieses Verfahren verfehlt jedoch naturgemäss seine Wirkung bei solchen Leichtsiedern, wie beispielsweise Ethylchlorid, die bei derartigen Chlorierungsreaktionen unverändert bleiben.

Aufgabe der Erfindung war es nun, die Aufarbeitungsprozedur für 1,2-Dichlorethan, das als Einsatzstoff für eine Pyrolyse verwendet werden soll, ohne Einbusse an Effizienz zu vereinfachen. Weiterhin war es Aufgabe der Erfindung, 1,2-Dichlorethan so aufzubereiten, dass es auch unter milden Bedingungen pyrolysiert werden kann.

Es wurde nun gefunden, dass die Anwesenheit von Ethylchlorid im 1,2-Dichlorethan, das unter milden Bedingungen pyrolysiert werden soll, keinen störenden Einfluss hat, wenn es lediglich in einer Konzentration von maximal 30 Gew.-ppm vorliegt. Überraschenderweise wird in diesem Konzentrationsbereich Ethylchlorid offenbar nicht, zumindest jedoch nicht mehr in störendem Ausmass, in die Polymerisationshemmer Allen, Methylacetylen und 1,3-Butadien übergeführt.

Gegenstand der Erfindung ist ein Verfahren zur Aufbereitung von 1,2-Dichlorethan für die Pyrolyse zu Vinylchlorid und Chlorwasserstoff, wobei

a) 1,2-Dichlorethan verwendet wird, das zu einem Anteil von 35 bis 55 Gew.-% solches 1,2-Dichlorethan enthält, das bei der Pyrolyse nicht umgesetzt und zur Pyrolyse zurückgefuhrt wurde,

b) die Pyrolyse bei Drücken von 10 bis 15 bar abs. und Temperaturen von 480 bis 540 °C durchgeführt wird,

c) 1,2-Dichlorethan vor Eintritt in die Spaltzone des Pyrolyseofens mittels Wärmetausch mit Wasserdampf von 16 bis 24 bar abs. vorverdampft wird,

d) das den Spaltofen verlassende Pyrolyseprodukt zunächst durch Abtrennen von Chlorwasserstoff und Vinylchlorid aufgearbeitet wird,

e) verbleibendes, nicht umgesetztes 1,2-Dichlorethan durch Umsetzen mit Chlor in Flüssigphase bei Temperaturen von 0 bis 80 °C in Gegenwart von hydroxylgruppenhaltigen Aromaten von Leichtsiedern befreit wird,

das dadurch gekennzeichnet ist, dass

f) das Produkt gemäss e) lediglich in einer solchen Teilmenge einer Destillation zur Abtrennung von Leichtsiedern unterworfen wird, dass der Ethylchloridgehalt im 1,2-Dichlorethan gemäss a) maximal 30 Gew.-ppm beträgt.

Das erfindungsgemässe Verfahren ist darauf zugeschnitten, die Aufarbeitungsprozedur für bei der Pyrolyse nicht umgesetztes, zur Pyrolyseeinheit wieder rückzuführendes 1,2-Dichlorethan zu verbessern, insbesondere die dabei erforderliche destillative Aufarbeitung energiesparender zu gestalten.

Im Rahmen der Erfindung wird 1,2-Dichlorethan pyrolysiert, das bezogen auf seine Gesamtmenge 35 bis 55 Gew.-% solches 1,2-Dichlorethan enthält, das bei der Pyrolyse nicht umgesetzt und wieder zur Pyrolyse zurückgeführt wird. Der restliche Anteil von 45 bis 65 Gew.-% kann an sich aus beliebigen Quellen stammen. In der Praxis wird überwiegend 1,2-Dichlorethan eingesetzt, das aus einer Direktchlorierung von Ethylen und/oder aus einer Oxichlorierung von Ethylen stammt.

Erfindungsgemäss darf der Anteil an Ethylchlorid in der Gesamtmenge des zu pyrolysierenden 1,2-Dichlorethans 30 Gew.-ppm nicht uberschreiten.

Das aufbereitete 1,2-Dichlorethan wird zunächst über einen mit Wasserdampf betriebenen externen Vorverdampfer in den gasförmigen Zustand übergeführt und mit einer Temperatur von 185 bis 205 °C der Pyrolyse aufgegeben, die bei 10 bis 15 bar abs. und einer Temperatur von 480 bis 540 °C erfolgt.

Die den Pyrolyseofen verlassenden Spaltgase werden zunächst auf eine Temperatur von 150 bis 250 °C abgeschreckt, danach weiter bis mindestens auf ihren Taupunkt abgekühlt und anschliessend einer Kolonne zur Abtrennung von Chlorwasserstoff aufgegeben. Das Sumpfprodukt der Chlorwasserstoff-Kolonne wird in eine weitere Kolonne geleitet, in der über Kopf Vinylchlorid abgezogen wird. Im Sumpf dieser Kolonne verbleibt im wesentlichen 1,2-Dichlorethan, das mit Leicht- und Hochsiedern verunreinigt ist.

Das Sumpfprodukt der Vinylchlorid-Kolonne wird nun nach Abkühlen auf eine Temperatur von 0 bis 80 °C einem Chlorierungs/Dechlorierungs-Verfahren unterzogen, um solche Leichtsieder, die sich unter den gegebenen Chlorierungsbedingungen in Hochsieder umwandeln lassen, zusammen mit ohnehin bereits gebildeten Hochsiedern abtrennen zu können. Es sind dies insbesondere 1-Chlorbutadien-1,3 und 2-Chlorbutadien-1,3, ferner geringere Mengen an Benzol, 1,1-Dichlorethylen und 1,2-Dichlorethylen. Ein derartiges Chlorierungs/Dechlorierungs-Verfahren ist gemäss EP-PS 2 501 bekannt. Dabei wird die 0 bis 80 °C aufweisende Mischung zunächst mit hydroxylgruppenhaltigen Aromaten der Art o-, m-Kresol bzw. deren Mono- oder Dichlorderivaten versetzt und anschliessend mit einer solchen Menge überschüssigen Chlors versetzt, dass nach Chlorierung maximal 800 Gew.-ppm an freiem Chlor in der Mischung verbleiben. Diese freie Chlormenge wird danach in einer zweiten Stufe durch Zugabe von äquimolaren Mengen an Ethylen entfernt. Das beschriebene Verfahren besitzt insbesondere den Vorteil, dass bei dieser Verfahrensweise kein weiteres Ethylchlorid als Nebenprodukt gebildet wird.

Mindestens 80 Gew.-% des die Dechlorierungsstufe verlassenden, Chlopren-freien Produktstroms werden unmittelbar einer Hochsiederkolonne aufgegeben, wo über Kopf 1,2-Dichlorethan abgezogen und schliesslich nach Vermischen mit 1,2-Dichlorethan aus anderen Quellen in die Pyrolyse rückgeführt wird. Maximal 20 Gew.-% des Produktstroms werden zunächst einer sauren Wäsche mit folgender Neutralisation unterzogen. Das feuchte Roh-Dichlorethan-Gemisch wird darauf einer Leichtsiederkolonne aufgegeben, wo über Kopf Leichtsieder und Wasser ausgeschleust werden. Das Sumpfprodukt der Leichtsiederkolonne wird schliesslich unter Vereinigung der Produktströme der Hochsiederkolonne aufgegeben.

Nach dem erfindungsgemässen Verfahren gelingt es, die Aufarbeitungsprozedur für bei einer Pyrolyse nicht umgesetzten 1,2-Dichlorethans wesentlich zu vereinfachen und insbesondere den problematischen Schritt der Leichtsiederabtrennung zu vereinfachen und energiesparender zu gestalten, ohne dabei sonstige verfahrenstechnische Nachteile in Kauf nehmen zu müssen. Es können mindestens 80 Gew.-% des bei der Pyrolyse nicht umgesetzten 1,2-Dichlorethans an einer Leichtsiederkolonne vorbeigeschleust werden, ohne dass sich dabei die in Kauf genommene Verunreinigung mit Ethylchlorid negativ auswirkt, obwohl bei einer Hochrechnung der aus 30 Gew.-ppm Ethylchlorid sich bildenden Nebenprodukte bereits nicht mehr akzeptable Mengen an Polymerisationshemmern im Vinylchlorid auftreten sollten.

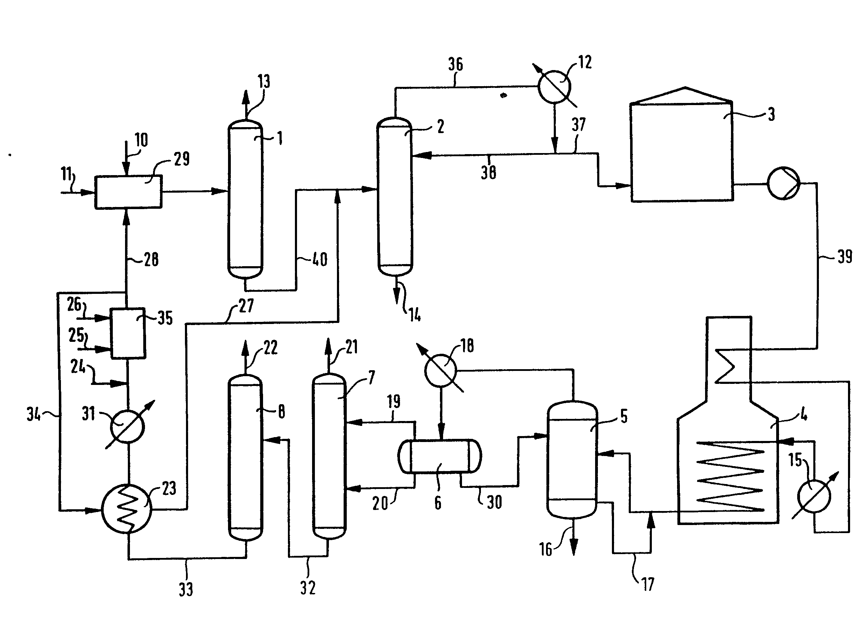

Die Abbildung 1 zeigt das Fliessschema einer bevorzugten Ausführungsform des Verfahrens. Die Abbildung wird im folgenden Beispiel näher erläutert:

Beispiel 1

In den Pyrolyseofen 4 wurden 74,4 t/h 1,2-Dichlorethans, verunreinigt mit 25 Gew.-ppm Ethylchlorid, aus dem Vorratsbehälter 3 nach Vorwärmung in der Konvektionszone des Spaltofens und Verdampfen im externen Verdampfer 15 bei einem Druck von 11 bar abs. und einer Temperatur von 506 °C zu 28 t/h Vinylchlorid und 16,4 t/h Chlorwasserstoff pyrolysiert, wobei, entsprechend einer Spaltrate von ca. 60% 29,76 t/h des zugeführten 1,2-Dichlorethans nicht umgesetzt blieben.

Das den Spaltofen 4 verlassende Reaktionsgemisch wurde durch Einspritzen von Sumpfprodukt aus der Quenschkolonne 5 über Leitung 17 auf 155 °C abgekühlt und der Quenschkolonne 5 aufgegeben. Dem Gemisch wurde innerhalb der Kolonne durch Wärmetausch mit Rücklauf, der über Leitung 30 eingeführt wurde, weitere Wärme entzogen. Die Brüden der Quenschkolonne wurden im Kondensator 18 weiter abgekühlt, wodurch Teilkondensation eintrat. Aus dem Sumpf der Quenschkolonne wurden bei 16 Teere und Hochsieder ausgeschleust.

Die bei 18 kondensierte flüssige Phase wurde im Sammler 6 aufgefangen und gelangte unter teilweiser Rücklaufführung zur Quenschkolonne 5. Die im Sammler 6 sich trennenden kondensierten und nichtkondensierten Bestandteile wurden separat über Leitung 19 und 20 der HCl-Kolonne 7 aufgegeben, wo am Kopf bei 21 16,4 t/h reinen Chlorwasserstoffs anfielen. Aus dem Sumpf der HCl-Kolonne 7 floss das verbleibende Gemisch aus Vinylchlorid und nicht umgesetztem 1,2-Dichlorethan über Leitung 32 zur Vinylchlorid-Kolonne 8, wo am Kopf bei 22 28 t/h Vinylchlorid mit einem Gehalt von 6,6 Gew.-ppm 1,3-Butadien gewonnen wurden.

Das Sumpfprodukt der Vinylchlorid-Kolonne 8, im wesentlichen nicht umgesetztes 1,2-Dichlorethan, wies einen Gehalt von 7,500 Gew.-ppm Benzol, 2,300 Gew.-ppm Chloropren, 230 Gew.-ppm 1-Chlorbutadien-1,3, 65 Gew.-ppm Ethylchlorid, 3,000 Gew.-ppm Tetrachlorkohlenstoff, 375 Gew.-ppm Chloroform und 750 Gew.-ppm 1,1-Dichlorethan auf.

Das 150 °C heisse Sumpfprodukt aus der Vinylchlorid-Kolonne 8 wurde über Leitung 33 in den Gegenstromwärmetauscher 23 geführt und auf 70 °C abgekühlt, wobei sich chloriertes/dechloriertes, nicht umgesetztes Pyrolyse-Dichlorethan auf etwa 110 °C vorwärmte. Der aus dem Gegenstromwärmetauscher 23 austretende Produktstrom wurde anschliessend im Wasserkühler 31 auf eine Temperatur von 30 °C gebracht. Nach Zugabe von 0,3 kg/h o-Kresol bei 24 wurde im Chlorierungs/Dechlorierungs-Reaktor durch Zugabe von 85,5 kg/h Chlor bei 25 und 6,5 kg/h Ethylen bei 26 der Chlorierung zugängliche Leichtsieder, im wesentlichen Chloropren in die entsprechenden hochsiedenden Chlorderivate umgewandelt.

25,36 t/h des von Chloropren befreiten, nicht umgesetzten Pyrolyse-Dichlorethans strömten über Leitung 34 und 27 direkt zur Hochsiederkolonne 2, wo am Kopf chloroprenfreies 1,2-Dichlorethan über Leitung 36 zum Kondensator 12 strömten. Das kondensierte 1,2-Dichlorethan wurde schliesslich unter teilweisem Rücklauf in die Kolonne über Leitung 38 durch Leitung 37 in den Vorratsbehälter 3 abgezogen.

Im Vorratsbehälter 3 lag gereinigtes 1,2-Dichlorethan, das über Leitung 39 in den Spaltofen 4 gepumpt wurde von folgender Zusammensetzung vor.

99,53 Gew.-% 1,2-Dichlorethan
0,0026 Gew.-% Ethylchlorid
0,30 Gew.-% Benzol
0,015 Gew.-% Chloroform
0,12 Gew.-% Tetrachlorkohlenstoff
0,03 Gew.-% 1,1-Dichlorethan

Das vorliegende Dichlorethan ergab sich aus einer Mischung aus 40 Gew.-% nicht umgesetzten Pyrolyse-Dichlorethans und 60 Gew.-% Dichlorethans aus einer Direkt- und Oxichlorierung.

Aus dem Sumpf der Hochsiederkolonne 2 wurden bei 14 Hochsieder abgezogen.

4,4 t/h des chloroprenfreien nicht umgesetzten Pyrolyse-Dichlorethans strömten über Leitung 28 zur Wäsche 29, wo gleichzeitig über 10 22 t/h rohes Dichlorethan aus einer Ethylendirektchlorierung und 23,2 t/h rohes Dichlorethan aus einer Ethylenoxichlorierung zusammen mit dem nicht umgesetzten Pyrolyse-Dichlorethan gewaschen und neutralisiert wurden. Anschliessend wurde das neutrale, feuchte Roh-Dichlorethangemisch der Azeotrop- bzw. Leichtsiederkolonne 1 aufgegeben, wo über Kopf bei 13 Wasser und Leichtsieder ausgeschleust wurden. Das Sumpfprodukt der Kolonne 1 gelangte frei von Leichtsiedern über Leitung 40 in die Hochsiederkolonne 2.

Der Wasserdampfverbrauch der Leichtsiederkolonne lag bei 10,7 t/h. Die Ofenlaufzeit betrug 10 Monate. Die Ausbeute an Vinylchlorid und Chlorwasserstoff betrug 99,4% bzw. 99,6%, bezogen auf den Gesamtverbrauch von 1,2-Dichlorethan.

Vergleichsbeispiel 1

Es wurde die Arbeitsweise gemäss Beispiel 1 wiederholt, mit der Abänderung, dass die Gesamtmenge an chloriertem/dechloriertem, nicht umgesetzten Pyrolyse-Dichlorethan über Leitung 28 zur Wäsche 29 und schliesslich zur Azeotrop- bzw. Leichtsiederkolonne 1 geleitet wurde. Das erhaltene 1,2-Dichlorethan war frei von Chloropren und Ethylchlorid.

Das durch Pyrolyse dieses Produkts erhaltene Vinylchlorid wies einen Gehalt von 6,5 Gew.-% 1,3-Butadien auf. Der spezifische Wasserdampfverbrauch zum Betrieb der Leichtsiederkolonne 1 lag bei 16,4 t/h gegenüber einem Wasserdampfverbrauch von lediglich 10,7 t/h bei der Arbeitsweise gemäss Beispiel 1.

Vergleichsbeispiel 2

Es wurde unter den Pyrolysebedingungen gemäss Beispiel 1 1,2-Dichlorethan pyrolysiert, das 50 Gew.-ppm Ethylchlorid enthielt. Das dabei erzeugte Vinylchlorid enthielt 20 Gew.-ppm 1,3-Butadien, 15 Gew.-ppm Methylchlorid, 5 Gew.-ppm Allen und 2 Gew.-ppm Methylacetylen.

**Patentanspruch**

Verfahren zur Aufbereitung von 1,2-Dichlorethan für die Pyrolyse zu Vinylchlorid und Chlorwasserstoff, wobei

a) 1,2-Dichlorethan verwendet wird, das zu einem Anteil von 35 bis 55 Gew.-% solches 1,2-Dichlorethan enthält, das bei der Pyrolyse nicht umgesetzt und zur Pyrolyse zurückgeführt wurde,

b) die Pyrolyse bei Drücken von 10 bis 15 bar abs. und Temperaturen von 480 bis 540 °C durchgeführt wird,

c) 1,2-Dichlorethan vor Eintritt in die Spaltzone des Pyrolyseofens mittels Wärmetausch mit Wasserdampf von 16 bis 24 bar abs. vorverdampft wird,

d) das den Spaltofen verlassende Pyrolyseprodukt zunächst durch Abtrennen von Chlorwasserstoff und Vinylchlorid aufgearbeitet wird,

e) verbleibendes, nicht umgesetztes 1,2-Dichlorethan durch Umsetzen mit Chlor in Flüssigphase bei Temperaturen von 0 bis 80 °C in Gegenwart von hydroxylgruppenhaltigen Aromaten von Leichtsiedern befreit wird, dadurch gekennzeichnet, dass

f) das Produkt gemäss e) lediglich in einer solchen Teilmenge einer Destillation zur Abtrennung von Leichtsiedern unterworfen wird, dass der Ethylchloridgehalt im 1,2-Dichlorethan gemäss a) maximal 30 Gew.-ppm beträgt.

**Revendication**

Procédé pour traiter du dichloro-1,2 éthane en vue de le purifier et de le préparer pour la pyrolyse donnant du chlorure de vinyle et du chlorure d'hyd... ...lé selon lequel:

a) ...ro-1,2 éthane qui con... ...e 35 à 55% en poids du ...nt pas réagi lors de la ...vers la pyrolyse,

b) ...ous des pressions ...et à des températures de ...à 540 °C,

c) avant ...rée dans la zone de scission du four de pyr... ...on effectue une évaporation préliminaire du dichloro-1,2 éthane, par échange de chaleur avec de la vapeur d'eau sous une pression absolue de 16 à 24 bars,

d) on traite tout d'abord, pour en séparer le chlorure d'hydrogène et le chlorure de vinyle, le produit de pyrolyse sortant du four de scission et de pyrolyse,

e) par réaction avec du chlore en phase liquide à des températures de 0 à 80 °C, en présence d'hydrocarbures aromatiques contenant des groupes hydroxyles, on débarrasse de ses constituants volatils le dichloro-1,2 éthane restant, n'ayant pas réagi, procédé caractérisé en ce que:

f) on soumet à une distillation, pour en séparer les constituants volatils à bas point d'ébullition, le produit selon e) tout simplement en une proportion telle que la teneur en chlorure d'éthyle dans le dichloro-1,2 éthane selon a) soit d'au maximum 30 ppm en poids.

**Claim**

Process for the pretreatment of 1,2-dichloroethane for its pyrolysis to vinyl chloride and hydrogen chloride, wherein

a) 1,2-dichloroethane which contains an amount of 35 to 55% by weight of 1,2-dichloroethane which was not reacted during the pyrolysis and has been recycled to the pyrolysis is used,

b) the pyrolysis is carried out under pressures of 10 to 15 bar absolute and at temperatures of 480 to 540 °C,

c) 1,2-dichloroethane is pre-vaporized by means of heat exchange with steam under 16 to 24 bar absolute before entry into the cleavage zone of the pyrolysis furnace,

d) the pyrolysis product which leaves the cleavage furnace is first worked up by removing the hydrogen chloride and vinyl chloride, and

e) unreacted 1,2-dichloroethane which remains is freed from low-boiling constituents by reaction with chlorine in the liquid phase at temperatures of 0 to 80 °C in the presence of aromatics containing hydroxyl groups, characterized in that

f) the product according to e) is subjected to distillation for removal of low-boiling constituents merely in a part amount such that the ethyl chloride content in the 1,2-dichloroethane according to a) is not more than 30 ppm by weight.

1/1